# EUROPEAN PATENT SPECIFICATION

(11) **EP 1 150 833 B1**
(45) Date of publication and mention of the grant of the patent: **05.04.2006**
(21) Application number: 00913349.7
(22) Date of filing: 02.02.2000
(51) Int. Cl.: B32B 5/02, D04H 13/00, A61F 13/15

(54) **ELASTIC LAMINATE AND DISPOSABLE ARTICLE INCLUDING SPUNBONDED NONWOVEN OF POLYPROPYLENE/POLYETHYLENE COPOLYMER**
ELASTISCHES LAMINAT UND WEGWERFARTIKEL, DIE EINEN SPINNVLIESSTOFF AUS POLYETHYLEN/POLYPROPYLEN COPOLYMERISATEN ENTHALTEN
STRATIFIE ELASTIQUE ET ARTICLE JETABLE COMPRENANT UN NON-TISSE FILE-LIE EN COPOLYMERE DE POLYPROPYLENE/POLYETHYLENE

(30) Priority: 02.02.1999 WO PCT/US99/02177; 02.02.1999 WO PCT/US99/02178
(43) Date of publication of application: 07.11.2001
(73) Proprietor: THE PROCTER & GAMBLE COMPANY, Cincinnati, Ohio 45202 (US)
(72) Inventor: TAO, Jie, Ashiya-City 659-0032 (JP); REZAI, Ebrahim, Kobe 658-0032 (JP)
(74) Representative: Kremer, Véronique Marie Joséphine
(86) International application number: PCT/US2000/002770
(87) International publication number: WO 2000/046023

(56) References cited:
- EP-A- 0 683 260
- EP-A- 0 693 585
- WO-A-96/10481
- WO-A-96/16216
- US-A- 5 607 798
- US-A- 5 652 051

## Description

### FIELD

The present invention relates to elastic laminates and disposable articles. More specifically, the present invention relates to elastic laminates and disposable articles which include a spunbonded nonwoven layer of a polypropylene/polyethylene copolymer. Examples of such disposable articles include sweat bands, bandages, body wraps, disposable underwears, disposable garments including disposable diapers (adult and baby) including pull-on diapers and training pants, disposable panties for menstrual use, and disposable absorbent pads including sanitary napkins and incontinence devices.

### BACKGROUND

Elastic laminates have previously been used in a variety of disposable products, including sweat bands, bandages, body wraps, disposable underwears, disposable garments including disposable diapers (adult and baby) including pull-on diapers and training pants, disposable panties for menstrual use, and disposable absorbent pads including sanitary napkins and incontinence devices. Herein, "elastic laminate" refers to an elastically stretchable two or more layered materials including at least one elastically stretchable single layer material. It is generally expected that these articles provide good fit to the body and/or skin of the user by using a suitable elastic laminate during the entire use period of products.

A "zero strain" stretch laminate is one type of elastic laminate which is preferably used for such disposable products. For example, methods for making "zero strain" stretch laminate webs are disclosed in U.S. Patent No. 5,167,897 issued to Weber et al. on December 1, 1992; U.S. Patent No. 5,156,793 issued to Buell et al. on October 20, 1990; and U.S. Patent No. 5,143,679 issued to Weber et al. on September 1, 1992. In a manufacturing process for such "zero strain" stretch laminate, an elastomeric material is operatively joined to at least one non-elastic component material in a substantially untensioned (zero strain) condition. At least a portion of the resultant non-elastic composite laminate is then subjected to mechanical stretching sufficient to permanently elongate the non-elastic component material. The composite stretch laminate is then allowed to return to its substantially untensioned condition. Thus, the elastic laminate is formed into a "zero strain" stretch laminate. Herein, "zero strain" stretch laminate refers to a laminate comprised of at least two plies of material which are secured to one another along at least a portion of their coextensive surfaces while in a substantially untensioned ("zero strain") condition; one of the plies comprising a material which is stretchable and elastomeric (i.e., will return substantially to its untensioned dimensions after an applied tensile force has been released) and a second ply which is elongatable (but not necessarily elastomeric) so that upon stretching the second ply will be, at least to a degree, permanently elongated so that upon release of the applied tensile forces, it will not fully return to its original undeformed configuration. The resulting stretch laminate is thereby rendered elastically extensible, at least up to the point of initial stretching, in the direction of initial stretching.

As is noted in the above, the manufacturing process of such "zero strain" stretch laminate includes the step of subjecting the non-elastic composite laminate to mechanical stretching sufficient to permanently elongate the non-elastic component material. This step is additional to normal elastic lamination processes and gives limitations to component materials to be used in the elastic laminate. For example, in the case where a nonwoven web is used in the elastic laminate as one of the component materials, it needs to have enough physical strength or toughness against the mechanical stretching applied since those materials tend to be mechanically damaged by the process. If the nonwoven web does not have enough physical strength or toughness and thus it is easily damaged, the resultant "zero strain" stretch laminate can not have an expected physical property, resulting in causing an undesirable break of the "zero strain" stretch laminate. For example, such a "zero strain" stretch laminate tends to be easily shred, torn or delaminated by the stress which is applied thereto during the mechanical stretch process and/or during the use of articles. Therefore, the nonwoven web to be used in the "zero strain" stretch laminate needs to be carefully selected.

Based on the foregoing, there is a need for a "zero strain" stretch laminate that includes a nonwoven layer having an increased physical strength or toughness. There is also a need for a disposable article that employs such an elastic laminate.

Infants and other incontinent individuals wear disposable pull-on garments such as disposable pull-on diapers to receive and contain urine and other body exudates. Disposable pull-on garments having fixed sides, which are also called "pant type" garments, have become popular for use on children able to walk and often who are toilet training. These pull-on garments have side panels with edges that are seamed together to form two leg openings and a waist opening. In order to contain body exudates as well as fit a wide variety of body shapes and sizes, these pull-on garments need to fit snugly about the waist and legs of the wearer without drooping, sagging or sliding down from its position on the torso. Examples of these pull-on garments are disclosed, for example, in U.S. Patent No. 5,171,239 issued to Igaue et al. on December 15; 1992; U.S. Patent No. 4,610,681 issued to Strohbeen et al. on September 9, 1986; U.S. Patent No. 4,940,464 issued to Van Gompel et al. on July 10, 1990; U.S. Patent No. 5,246,433 to issued Hasse et al. on September 21, 1993; U.S. Patent No. 5,569,234 issued to Buell et al. on October 29, 1996; and WO 96/31176 (Ashton) published on October 10, 1996.

In many disposable pull-on garments, the seams of the side panels are formed by an application of pressure and heat to edge portions of the side panels to be seamed. For example, WO 98/22285 (Schmitz) published on May 28, 1998 discloses such a seaming method. The application of pressure and heat causes a melt of component sheet materials used in the side panels, in particular in the contacting surface materials of the side panels thereby forming a necessary bond between the side panels.

To get a strong seam, it is generally preferred that the difference of the melting points between the two contacting surface materials to be seamed is small. However, if different types of materials are employed in the contacting surfaces of the side panels, the heating needs to be carefully adjusted and controlled in the seaming process. Otherwise, one of the materials which has a relatively lower melting point tends to be melt much more than the other material which has a relatively higher melting point. (For example, if a polypropylene which has a melting point of about 160 °C is employed in one surface material and a polyethylene which has a melting point of about 125 °C in the other surface material, the difference of the melting points between the two contacting surface materials is about 35 °C). Such a difference in the melting points tends to result in forming a hole(s) in the seamed portion and/or a hard portion of melted material after its solidification. Since the formation of such a hole(s) tends to decrease the seaming strength, it needs to be avoided. The formation of such a hard portion after its solidification also needs to be avoided since it tends to cause a graze on the wearer's skin resulting in a red marking on the skin.

Based on the foregoing, there is a need for two contacting materials to be seamed in disposable pull-on garments that can provide an improved seam strength. There is also a need for two contacting materials to be seamed in disposable pull-on garments that does not form a hard portion of melted material in the seamed portion after its solidification.

### SUMMARY

The present invention is directed to an elastic laminate which is elastically extensible in at least one direction. The elastic laminate includes an elastic member having a first surface and a second surface opposing the first surface; and a spunbonded nonwoven layer joined to the first surface of the elastic member to form a laminate. The spunbonded nonwoven layer includes component fibers formed by a polypropylene/polyethylene copolymer. The laminate is mechanically stretched to form an elastic laminate.

The present invention is also directed to a disposable article having the elastic laminate.

The foregoing answers the need for a "zero strain" stretch laminate that includes a nonwoven layer having an increased physical strength or toughness, and the need for a disposable article that employs such an elastic laminate.

The foregoing further answers the need for two contacting materials to be seamed in disposable pull-on garments that can provide an improved seam strength, and/or a need for two contacting materials to be seamed in disposable pull-on garments that does not form a hard portion of melted material in the seamed portion after its solidification.

These and other features, aspects, and advantages of the present invention will become evident to those skilled in the art from reading of the present disclosure.

### BRIEF DESCRIPTION OF THE DRAWINGS

While the specification concludes with claims particularly pointing out and distinctly claiming the invention, it is believed that the invention will be better understood from the following description of preferred embodiments which is taken in conjunction with the accompanying drawings and which like designations are used to designate substantially identical elements, and in which:
Fig. 1 is a perspective view of one preferred embodiment of the disposable pull-on garment of the present invention in a typical in use configuration;
Fig. 2 is a simplified plan view of the embodiment shown in Fig. 1 in its flat uncontracted condition showing the body-facing side the garment;
Fig. 3 is a cross-sectional view of a preferred embodiment taken along the section line 3-3 of Fig. 2;
Fig. 4 is a cross-sectional view of an elastic member (or an elastic laminate) of a preferred embodiment; and
Fig. 5 is a fragmentary enlarged perspective illustration of one preferred example of the elastomeric material layer.

### DETAILED DESCRIPTION

Herein, "comprise" means that other element(s) and step(s) which do not affect the end result can be added. These terms encompass the terms "consisting of" and "consisting essentially of".

Herein, "pull-on garment" refers to articles of wear which have a defined waist opening and a pair of leg openings and which are pulled onto the body of the wearer by inserting the legs into the leg openings and pulling the article up over the waist.

Herein, "disposable" describes garments which are not intended to be laundered or otherwise restored or reused as a garment (i.e., they are intended to be discarded after a single use and, preferably, to be recycled, composted or otherwise disposed of in an environmentally compatible manner).

Herein, "pull-on diaper" refers to pull-on garments generally worn by infants and other incontinent individuals to absorb and contain urine and feces. It should be understood, however, that the present invention is also applicable to other pull-on garments such as training pants, incontinent briefs, feminine hygiene garments or panties, and the like.

Herein, "panel" denotes an area or element of the pull-on garment. (While a panel is typically a distinct area or element, a panel may coincide (functionally correspond) somewhat with an adjacent panel.)

Herein, "layer" does not necessarily limit the element to a single strata of material in that a layer may actually comprise laminates or combinations of sheets or webs of the requisite type of materials.

Herein, "joined" or "joining" encompasses configurations whereby an element is directly secured to another by affixing the element directly to the other element, and configurations whereby the element is indirectly secured to the other element by affixing the element to intermediate member(s) which in turn are affixed to the other element.

Herein, "uncontracted state" is used to describe states of pull-on garments in its unseamed (i.e., seams are removed), flat and relaxed condition wherein all elastic materials used are removed therefrom.

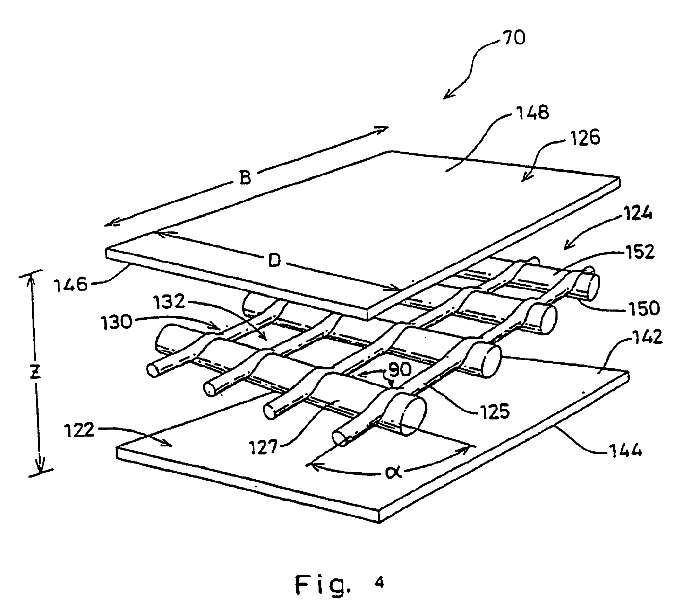

The elastic laminate of the present invention includes an elastic member and a spunbonded nonwoven layer joined to the elastic member. The elastic member of the present invention may take on a number of different configurations and materials. For example, the elastic member may be formed from one or a plurality of elastomeric material layers. Preferably, the elastic member is an elastic laminate or an elastic composite member which are formed by a plurality of material layers including at least one elastomeric material layer. In a preferred embodiment, the elastic member includes a laminate of an elastomeric material layer, a nonwoven coverstock layer joined to one surface of the elastomeric material layer, and more preferably a second nonwoven coverstock layer joined to the other surface of the elastomeric material layer. A more preferred example of the elastic member will be described in more detail hereinafter by referring to Fig. 4.

The elastomeric material layer may be formed in a wide variety of sizes, forms and shapes. In a preferred embodiment, the elastomeric material layer is in the form of a continuous plane layer. Preferred forms of a continuous plane layer include a scrim, a perforated (or apertures formed) film, an elastomeric woven or nonwoven, an elastomeric foam, and the like. The continuous plane layer may take any shape which can be suitably provided in products. Preferred shapes of a continuous plane layer include a quadrilateral including a rectangle and a square, a trapezoid, and the other polygons. In an alternative embodiment, the elastomeric material layer is in the form of discrete strands (or strings) which are not connected each other.

The elastomeric material may include all suitable elastic materials known in the art. Elastomeric materials suitable for use herein include synthetic or natural rubber materials known in the art. Preferred elastomeric materials include the diblock and triblock copolymers based on polystyrene and unsaturated or fully hydrogenerated rubber bolcks, and their blends with other polymers such as polyolefin polymers.

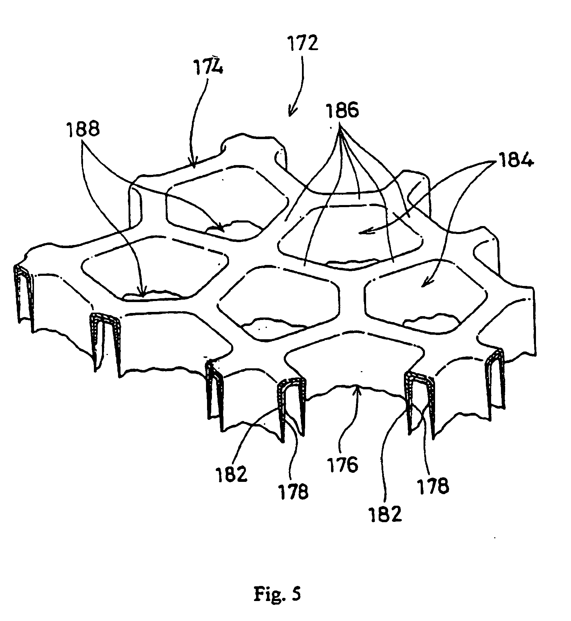

In one preferred embodiment, the elastomeric material layer is a porous, macroscopically-expanded, three-dimensional elastomeric web 172 as shown in Fig. 5. The web 172 has a continuous first surface 174 and a discontinuous second surface 176 opposing the first surface 174. The elastomeric web 172 preferably comprises a formed film interconnecting member 186 including at least two polymeric layers 178 and 182. The first layer 178 is substantially elastic and the second layer 182 is substantially less elastic than the first layer 178. At least one of the two polymeric layers 178 and 182 is formed from a polystyrene thermoplastic elastomer. The elastomeric web 172 exhibits a multiplicity of primary apertures 184 in the first surface 174 of the web 172. The primary apertures 184 are defined in the plane of the first surface 174 by a continuous network of the interconnecting member 186. The interconnecting member 186 exhibits an upwardly concave-shaped cross-section along its length. The interconnecting member 186 also forms secondary apertures 188 in the plane of the second surface 176 of the web 172. The apertures 184 and 188 may take any shape. A preferred elastomeric web is disclosed in International Publication No. WO 98/3716 (Curro et al.), published on August 27, 1998. A preferred porous elastomeric material for the elastomeric material layer is available from Tredegar Film Products under the designation X-25007.

In a more preferred embodiment, the elastomeric material layer is an elastomeric scrim which will be described in detail hereinafter.

The elastic member of the present invention has a first surface and a second surface opposing the first surface. The spunbonded nonwoven layer is joined to the first surface of the elastic member to form a laminate. The spunbonded nonwoven layer of the present invention includes component fibers formed by a polypropylene/polyethylene copolymer The laminate is mechanically stretched to form an elastic laminate (e.g., "zero strain" elastic laminate). The spunbonded nonwoven layer has a Young's modulus of greater than 4000 gf/inch (1575 N/cm). The spunbonded nonwoven layer has a stress of less than 1000 gf/inch (394 N/cm) at peak in the Stress-Strain curve. These and other preferred embodiments will be described in detail hereinafter.

By employing the spunbonded nonwoven layer of polypropylene/polyethylene copolymer in the elastic laminate, the elastic laminate of the present invention provides improved physical strength or toughness against the mechanical stretch applied thereto during the mechanical stretch process and/or during the use of articles. Consequently, the elastic laminate of the present invention can prevent an undesirable break of the elastic laminate, such as shredding and/or tearing.

The spunbonded nonwoven layer of the present invention can be formed by any spunbonded nonwoven process known in the art, for example, U.S. Patent No. 3,692,618 issued to Dorschner et al. on September 19, 1972; U.S. Patent No. 3,338,992 issued to Kinney on August 29, 1967; U.S. Patent No. 3,341,394 issued to Kinney on September 12, 1967; U.S. Patent No. 3,502,538 issued to Petersen on March 24, 1970; U.S. Patent No. 3,502,763 issued to Hartmann on March 24, 1970; U.S. Patent No. 3,909,009 issued to Cvetko et al. on September 30, 1975; U.S. Patent No. 3,542,615 issued to Dobo et al. on November 24, 1970; and U.S. Patent No. 4,340,563 issued to Appel et al. on July 20, 1982.

The polypropylene/polyethylene copolymer which forms the component fibers can be produced by copolymerizing from 0.5% to 20% by weight of polyethylene in the backbone of a polypropylene. Preferably, the polypropylene copolymer is a block copolymer produced by copolymerizing the polyethylene by block in the backbone of polypropylene. More preferably, the polypropylene/polyethylene copolymer is a random copolymer produced by copolymerizing the polyethylene randomly in the backbone of polypropylene.

Preferably, from 0.5% to 20%, more preferably from 5% to 15% by weight of polyethylene is contained by copolymerization in the backbone of polypropylene. A preferred polypropylene/polyethylene is produced when 7% by weight of polyethylene is copolymerized with the polypropylene.

Any type of polyethylene can be employed in the copolymerization. Preferred polyethylene includes a low density polyethylene (LDPE), a high density polyethylene (HDPE) and a linear low density polyethylene (LLDPE). A preferred polyethylene is a LLDPE. In a preferred embodiment, the polyethylene has an α olefin structure such as 1-hexene, 1-butene and 1-octene.

In a preferred embodiment, the spunbonded nonwoven layer has a basis weight of less than 35 g/m², and comprises component fibers having an average fiber denier of less than 2.5. Preferably, for products such as disposable garment and the like, the spunbonded nonwoven layer has a basis weight of from 15 g/m² to 30 g/m², more preferably from 17 g/m² to 25 g/m², and an average fiber denier of less than 1.9, and more preferably less than 1.5.

The polypropylene/polyethylene copolymer is less crystalline and thus has a broader melting point range than polypropylene itself. Preferably, the polypropylene/polyethylene copolymer has a melting point range between about 100 °C and 180 °C, more preferably between about 125 °C and 165 °C.

In a preferred embodiment, the spunbonded nonwoven layer has an embossment pattern formed by a number of discrete bonding spots or areas. Any embossment process known in the art can be used for forming such an embossment pattern in spunbonded nonwoven layer. For example, a preferred embossment pattern is formed by feeding a spunbonded nonwoven web through two bonding rolls which have a raised pattern such as spots or grids on the surface of the bonding rolls. The bonding rolls are heated to a softening temperature of the material of the component fibers (i.e., the polypropylene/polyethylene copolymer). The spunbonded nonwoven web passes between the heated bonding rolls so that the spunbonded nonwoven web can be compressed and heated by the bonding rolls in accordance with the pattern on the rolls, thereby forming an embossment pattern of discrete bonding spots or areas in the spunbonded nonwoven web. The resulting spunbonded nonwoven web is preferably used as the spunbonded nonwoven layer. As is well known in the art, the embossment holds the component fibers together and imparts an integrity to the nonwoven web or layer by bonding the component fibers within the nonwoven web or layer.

A preferred embossment pattern has lozenge shaped pin bonding areas wherein each pin has an average dimension of 0.65 x 0.74 mm, a vertical spacing of 1.23 mm between pins, and a horizontal spacing of 1.26 mm between pins. Preferably, the embossment pattern has an embossment area (or a bonded area) ratio of less than 25 %. In a preferred embodiment, the embossment area ratio of the nonwoven web or layer is from 5% to 20%, and more preferably from 10% to 18%.

The spunbonded nonwoven layer has a Young's modulus of greater than 4000 gf/inch (1575 N/cm), preferably greater than 5000 N/inch (1969 N/cm), and more preferably from 5000 gf/inch (1969 N/cm) to 12000 gf/inch (4724 N/cm).

The spunbonded nonwoven layer has a stress of less than 1000 gf/inch (394 N/cm) at peak in the Stress-Strain curve. Preferably, the spunbonded nonwoven layer has a stress of greater than 600 gf/inch (236 N/cm), more preferably from 700 gf/inch (276 N/cm) to 1000 gf/inch (394 N/cm) at peak in the Stress-Strain curve.

A preferred spunbonded nonwoven web which is used for the spunbonded nonwoven layer of polypropylene/polyethylene copolymer is available from Mitsui Chemical Co., Ltd., Tokyo, Japan, under Code No. 23GSM. This spunbonded nonwoven web includes component fibers formed by a random polypropylene/polyethylene copolymer which has about 7% by weight of polyethylene (LLPE) in the backbone of polypropylene. This spunbonded nonwoven web has a basis weight of about 20 g/m², and includes component fibers having an average fiber denier of about 20. This spunbonded nonwoven web has an average embossment ratio of about 18%. The polypropylene/polyethylene copolymer of this spunbonded nonwoven web has a melting point range between about 105 °C and 170 °C. This spunbonded nonwoven web has a Young's modulus of greater than 4000 gf/inch (1575 N/cm), and also has a stress of less than 1000 gf/inch (394 N/cm) at peak in the Stress-Strain curve.

The spunbonded nonwoven layer of the present invention is joined to the first surface of the elastic member by attachment means such as those well known in the art. For example, the spunbonded nonwoven layer may be secured to the first surface of the elastic member by a uniform continuous layer of adhesive, a patterned layer of adhesive, or an array of separate lines, spirals, or spots of adhesive. Alternatively, the attachment means may comprise heat bonds, pressure bonds, ultrasonic bonds, dynamic mechanical bonds, or any other suitable attachment means or combinations of these attachment means as are known in the art.

After the spunbonded nonwoven layer of the present invention is joined to the elastic member, at least a portion of the resultant laminate is then subjected to mechanical stretching sufficient to permanently elongate the spunbonded nonwoven layer. The laminate is then allowed to return to its substantially untensioned condition. The laminate is thus formed into a "zero strain" stretch laminate.

The present invention can be applied to a variety of disposable articles in need of a "zero strain" stretch laminate that includes a nonwoven layer having an increased physical strength or toughness. Preferred disposable articles include sweat bands, bandages, body wraps, disposable underwears, disposable garments including disposable diapers (adult and baby) including pull-on diapers and training pants, disposable panties for menstrual use, and disposable absorbent pads including sanitary napkins and incontinence devices. In a preferred embodiment, the disposable article is a disposable pull-on garment.

In another aspect of the invention, the present invention can also be applied to a variety of disposable pull-on garments in need of ear panels to be seamed with an improved seam strength. Preferred disposable pull-on garments include disposable pull-on underwears, disposable pull-on diapers (adult and baby) including training pants, and disposable panties for menstrual use.

In the following, preferred embodiments of a disposable pull-on garment will be described in detail by referring to drawings.

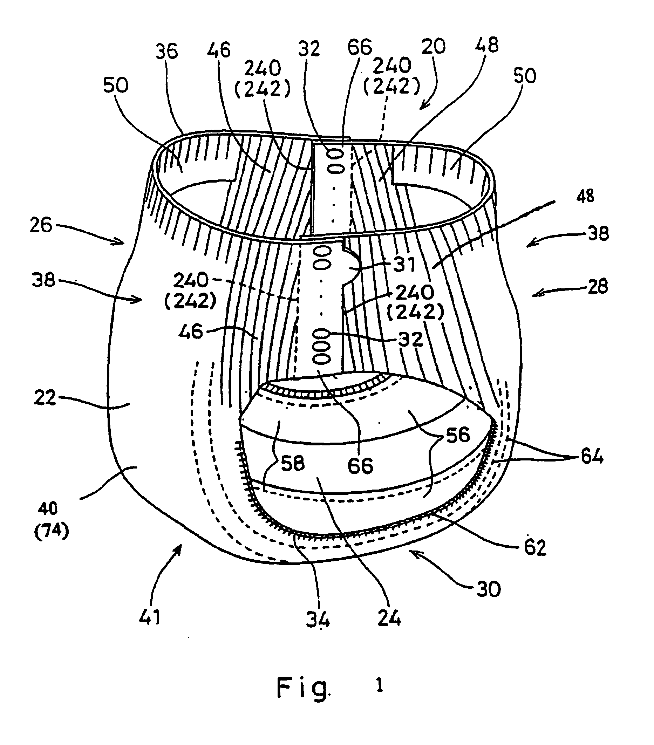

Fig. 1 shows one preferred embodiment of a disposable pull-on garment of the present invention (e.g., a unitary disposable pull-on diaper). Referring to Fig. 1, the disposable pull-on garment 120 has a front region 26; a back region 28 and a crotch region 30 between the front region 26 and the back region 28. A chassis 41 is provided in the front, back and crotch regions 26, 28 and 30. The chassis 41 includes a liquid pervious topsheet 24, a liquid impervious backsheet 22 associated with the topsheet 24, and an absorbent core 25 (not shown in Fig. 1) disposed between the topsheet 24 and the backsheet 22. The chassis 41 has side edges 220 which form edge lines 222 in the front region 26.

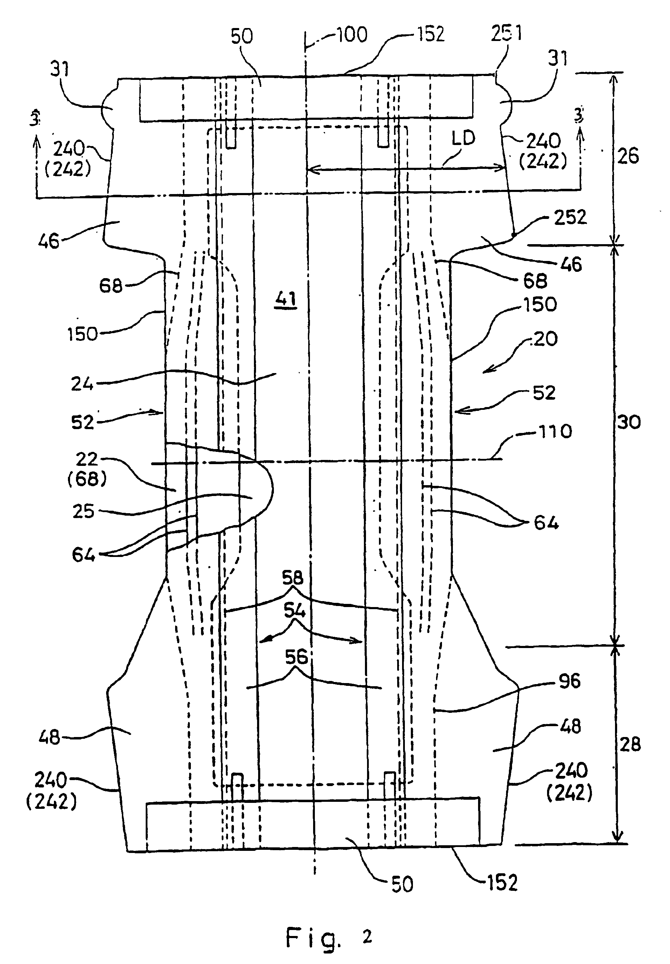

The disposable pull-on garment 20 further includes a pair of front ear panels 46 each extending laterally outward from the corresponding sides of the chassis 41 in the front region 26, and a pair of extensible back ear panels 48 each extending laterally outward from the corresponding sides of the chassis 41 in the back region 28. Each of the ear panels 46 and 48 has an outermost edge 240 which forms an outermost edge line 242. At least one of the outermost edge lines 242 has a nonuniform lateral distance LD from the longitudinal center line 100 (not shown in Fig. 1 but in Fig. 2) in the uncontracted state of the garment 20. The pull-on garment 20 further includes seams 32 each joining the front and back ear panels 46 and 48 along the corresponding edge lines 242 to form the two leg openings 34 and the waist opening 36.

In preferred embodiments, the pull-on garment 20 includes a chassis layer 40 which generally determines the overall shape of the pull-on garment 20. In the embodiment shown in Fig. 1, the chassis layer 40 is an outer cover nonwoven layer 74 which covers all of the outer-facing surface of the pull-on garment 20 to provide the feel and appearance of a cloth garment. Preferably, the outer cover nonwoven layer 74 is a continuous sheet or web formed by the spunbonded nonwoven of polypropylene/polyethylene copolymer of the present invention. The continuous sheet (i.e., the outer cover nonwoven layer 74) defines the front region 26, the back region 28 and the crotch region 30 between the front region 26 and the back region 28. Each of the ear panels 46 and 48 includes a portion of the chassis layer 40. Preferred pull-on garments which includes such a continuous sheet are disclosed in U.S. Patent No. 5,569,234 issued to Buell et al. on October 29, 1996.

In a preferred embodiment, at least one of, more preferably both of, the pairs of the ear panels 46 and 48 are elastically extensible in at least the lateral direction. In alternative embodiments, the ear panels 46 and 48 are elastically extensible both in the lateral and longitudinal directions. Herein, "extensible" refers to materials that are capable of extending in at least one direction to a certain degree without undue rupture. Herein, "elasticity" and "elastically extensible" refer to extensible materials that have the ability to return to approximately their original dimensions after the force that extended the material is removed. Herein, any material or element described as "extensible" may also be elastically extensible unless otherwise provided. The extensible ear panels 46 and 48 provide a more comfortable and contouring fit by initially conformably fitting the pull-on garment to the wearer and sustaining this fit throughout the time of wear well past when the pull-on garment has been loaded with exudates since the ear panels 46 and/or 48 allow the sides of the pull-on garment to expand and contract.

The ear panels 46 and 48 may be formed by unitary elements of the pull-on garment 20 (i.e., they are not separately manipulative elements secured to the pull-on garment 20, but rather are formed from and are extensions of one or more of the various layers of the pull-on garment). In a preferred embodiment, the ear panels 46 and 48 include at least one unitary element or a continuous sheet (e.g. the chassis layer 40) that forms a part of the chassis 41 and continuously extends into the ear panels 46 and 48. Alternatively, the ear panels 46 and 48 may only include discrete members (not shown in Figs.) which do not have any unitary element that also forms a part of the chassis 41. Such an ear panel structure may be formed by joining the discrete members to the corresponding sides of the chassis 41.

In a preferred embodiment, the pull-on garment 20 further includes seam panels 66 each extending laterally outward from each of the ear panels 46 and 48; and tear open tabs 31 each extending laterally outward from the seam panel 66. In a preferred embodiment, each of the seam panels 66 is an extension of the corresponding ear panels 46 and 48, or at least one of the component elements used therein (e.g., the chassis layer 40), or any other combination of the elements. More preferably, each of the tear open tabs 31 is also an extension of the corresponding seam panel 66 or at least one of its component elements used therein (e.g., the chassis layer 40), or any other combination of its elements.

In a preferred embodiment, the corresponding edge portions of the ear panels 46 and 48 are joined through the seam panels 66 in an overlaping manner to make an overlapped seam structure as shown in Fig. 1. Alternatively, the front and ear panels 46 and 48 can be seamed in a butt seam manner (not shown in Figs.). The bonding of the seams 32 can be performed by any suitable means known in the art appropriate for the specific materials employed in the chassis 41 and/or the ear panels 46 and 48. Thus, sonic sealing, heat sealing, pressure bonding, adhesive or cohesive bonding, sewing, autogeneous bonding, and the like may be appropriate techniques. Preferably, the seam panels 66 are joined by a predetermined pattern of heat/pressure or ultrasonic welds which withstands the forces and stresses generated on the garment 20 during wear. A preferred method for making the seams is disclosed in WO 98/22285 (Schmitz) published on May 28, 1998.

A continuous belt 38 is formed by the ear panels 46 and 48, and a part of the chassis 41 about the waist opening 36 as shown in Fig. 1. Preferably, elasticized waist bands 50 are provided in both the front region 26 and the back region 28. The continuous belt 38 acts to dynamically create fitment forces in the pull-on garment 20 when positioned on the wearer, to maintain the pull-on garment 20 on the wearer even when loaded with body exudates thus keeping the absorbent core 25 (not shown in Fig. 1) in close proximity to the wearer, and to distribute the forces dynamically generated during wear about the waist thereby providing supplemental support for the absorbent core 25 without binding or bunching the absorbent core 25.

Fig. 2 is a partially cut-away plan view of the pull-on garment 20 of Fig. 1 in its uncontracted state (except in the ear panels 46 and 48 which are left in their relaxed condition) with the topsheet 24 facing the viewer, prior to the ear panels 46 and 48 being joined together by the seams 32. The pull-on garment 20 has the front region 26, the back region 28 opposed to the front region 26, the crotch region 30 positioned between the front region 26 and the back region 28, and a periphery which is defined by the outer perimeter or edges of the pull-on garment 20 in which the side edges are designated 115 and 240, and the end edges or waist edges are designated 152. The topsheet 24 has the body-facing surface of the pull-on garment 20 which is positioned adjacent to the wearer's body during use. The backsheet 22 has the outer-facing surface of the pull-on garment 20 which is positioned away from the wearer's body. The pull-on garment 20 includes the chassis 41 including the liquid pervious topsheet 24, the liquid impervious backsheet 22 associated with the topsheet 24, and the absorbent core 25 positioned between the topsheet 24 and the backsheet 22. The garment 20 further includes the front and back ear panels 46 and 48 extending laterally outward from the chassis 41, the elasticized leg cuffs 52, and the elasticized waistbands 50. The topsheet 24 and the backsheet 22 have length and width dimensions generally larger than those of the absorbent core 25. The topsheet 24 and the backsheet 22 extend beyond the edges of the absorbent core 25 to thereby form the side edges 115 and the waist edges 152 of the garment 20. The liquid impervious backsheet 22 preferably includes a liquid impervious plastic film 68.

The pull-on garment 20 also has two centerlines, a longitudinal centerline 100 and a transverse centerline 110. Herein, "longitudinal" refers to a line, axis, or direction in the plane of the pull-on garment 20 that is generally aligned with (e.g. approximately parallel with) a vertical plane which bisects a standing wearer into left and right halves when the pull-on garment 20 is worn. Herein, "transverse" and "lateral" are interchangeable and refer to a line, axis or direction which lies within the plane of the pull-on garment that is generally perpendicular to the longitudinal direction (which divides the wearer into front and back body halves). The pull-on garment 20 and component materials thereof also have a body-facing surface which faces the skin of wearer in use and an outer-facing surface which is the opposite surface to the body-facing surface.

While the topsheet 24, the backsheet 22, and the absorbent core 25 may be assembled in a variety of well known configurations, exemplary chassis configurations are described generally in U.S. Patent 3,860,003 entitled "Contractible Side Portions for Disposable Diaper" which issued to Kenneth B. Buell on January 14, 1975; and U.S. Patent 5,151,092 entitled "Absorbent Article With Dynamic Elastic Waist Feature Having A Predisposed Resilient Flexural Hinge" which issued to Kenneth B. Buell et al., on September 29, 1992.

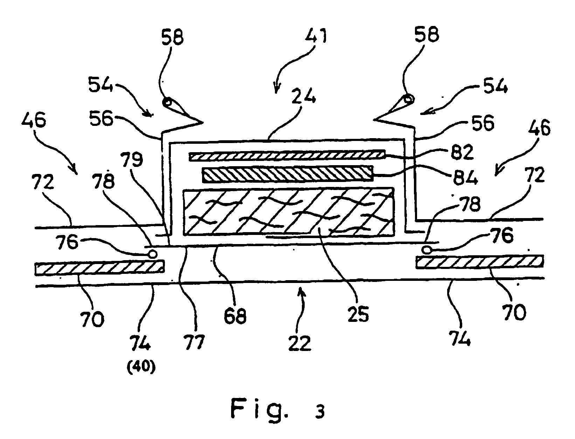

Fig. 3 is a cross-sectional view of a preferred embodiment taken along the section line 3-3 of Fig. 2. The pull-on garment 20 includes the chassis 41 including the liquid pervious topsheet 24, the liquid impervious backsheet 22 associated with the topsheet 24, and the absorbent core 25 positioned between the topsheet 24 and the backsheet 22. The pull-on garment further includes the front ear panels 46 each extending laterally outward from the chassis 41, and an inner barrier cuffs 54. Although Fig. 3 depicts only the structure of the front ear panel 46 and the chassis 41 in the front region 26, preferably a similar structure is also provided in the back region 28. In a preferred embodiment, each of the front ear panels 46 is formed by a lamination of an extended part of the barrier flap 56, an elastic member 70 and the outer cover nonwoven layer 74. Preferably, the elastic member 70 is formed by the elastic laminate of the present invention. The elastic member 70 includes a plane elastomeric material layer 124 (not shown in Fig. 3). Herein, "plane elastomeric material" refers to elastomeric materials which continuously extend in two dimensional directions. Preferred plane elastomeric materials include a scrim, a perforated (or apertures formed) film, an elastomeric woven or nonwoven, an elastomeric foam and the like. In a preferred embodiment, the plane elastomeric material layer 124 includes at least a portion that has a nonuniform lateral width.

In a preferred embodiment, the flap nonwoven web 72 is formed by a polypropylene based polymer. Preferably, the flap nonwoven web 72 is a spunbonded nonwoven web including component fibers of 100% of polypropylene. A preferred spunbonded nonwoven web which is suitably applied to the flap nonwoven web 72 is available as Code No. DAPP-35GSM from Fiberweb France, Inc. (Ziest, France). The outer cover nonwoven layer 74 is the spunbonded nonwoven of polypropylene/polyethylene copolymer of the present invention. Thus, when the corresponding ear panels 46 and 48 are joined in an overlapping manner, the flap nonwoven web 72 and the outer cover nonwoven layer 74 in the seam panels 66 are seamed (or heat bonded) together to form the seams 32. Since the flap nonwoven web 72 and the outer cover nonwoven layer 74 can be seamed at a seaming temperature which is close to the melting points of the flap nonwoven web 72 and the outer cover nonwoven layer 74, the formation a hole(s) in the seamed portion and/or a hard portion of melted material after its solidification can be prevented.

The absorbent core 25 can be any absorbent member which is generally compressible, conformable, non-irritating to the wearer's skin, and capable of absorbing and retaining liquids such as urine and other certain body exudates. The absorbent core 25 may be manufactured in a wide variety of sizes and shapes (e.g., rectangular, hourglass. "T"-shaped, asymmetric, etc.) and from a wide variety of liquid-absorbent materials commonly used in disposable pull-on garments and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 25 may vary (e.g., the absorbent core 25 may have varying caliper zones, a hydrophilic gradient, a superabsorbent gradient, or lower average density and lower average basis weight acquisition zones; or may include one or more layers or structures). Further, the size and absorbent capacity of the absorbent core 25 may also be varied to accommodate wearers ranging from infants through adults. However, the total absorbent capacity of the absorbent core 25 should be compatible with the design loading and the intended use of the garment 20.

A preferred embodiment of the garment 20 has an asymmetric, modified hourglass-shaped absorbent core 25 having ears in the front and back waist regions 26 and 28. Other exemplary absorbent structures for use as the absorbent core 25 that have achieved wide acceptance and commercial success are described in U.S. Patent No. 4,610,678 entitled "High-Density Absorbent Structures" issued to Weisman et al. on September 9, 1986; U.S. Patent No. 4,673,402 entitled "Absorbent Articles With Dual-Layered Cores" issued to Weisman et al. on June 16, 1987; U.S. Patent No. 4,888,231 entitled "Absorbent Core Having A Dusting Layer" issued to Angstadt on December 19, 1989; and U.S. Patent No. 4,834,735, entitled "High Density Absorbent Members Having Lower Density and Lower Basis Weight Acquisition Zones", issued to Alemany et al. on May 30, 1989.

The chassis 41 may further include an acquisition/distribution core 84 of chemically stiffened fibers positioned over the absorbent core 25, thereby forming a dual core system. In a preferred embodiment, the fibers are hydrophilic chemically stiffened cellulosic fibers. Herein, "chemically stiffened fibers" means any fibers which have been stiffened by chemical means to increase stiffness of the fibers under both dry and aqueous conditions. Such means include the addition of chemical stiffening agents which, for example, coat and/or impregnate the fibers. Such means also include the stiffening of the fibers by altering the chemical structure of the fibers themselves, e.g., by cross-linking polymer chains.

The fibers utilized in the acquisition/distribution core 84 can also be stiffened by means of chemical reaction. For example, crosslinking agents can be applied to the fibers which, subsequent to application, are caused to chemically form intrafiber crosslink bonds. These crosslink bonds can increase stiffness of the fibers. Whereas the utilization of intrafiber crosslink bonds to chemically stiffen the fibers is preferred, it is not meant to exclude other types of reactions for chemical stiffening of the fibers.

In the more preferred stiffened fibers, chemical processing includes intrafiber crosslinking with crosslinking agents while such fibers are in a relatively dehydrated, defibrated (i.e. individualized), twisted, curled condition. Suitable chemical stiffening agents include monomeric crosslinking agents including, but not limited to, C₂-C₈ dialdehydes and C₂-C₈ monoaldehydes having an acid functionality can be employed to form the cosslinking solution. These compounds are capable of reacting with at least two hydroxyl groups in a single cellulose chain or on proximately located cellulose chains in a single fiber. Such crosslinking agents contemplated for use in preparing the stiffened cellulose fibers include, but are not limited to, glutaraldehyde, glyoxal, formaldehyde, and glyoxylic acid. Other suitable stiffening agents are polycarboxylates, such as citric acid. The polycarboxylic stiffening agents and a process for making stiffened fibers from them are described in U.S. Patent No. 5,190,563, entitled "Process for Preparing Individualized, Polycarboxylic Acid crosslinked Fibers" issued to Herron, on March 2, 1993. The effect of crosslinking under these conditions is to form fibers which are stiffened and which tend to retain their twisted, curled configuration during use in the absorbent articles herein. Such fibers, and processes for making them are cited in the above incorporated patents.

Preferred dual core systems are disclosed in U.S. Patent No. 5,234,423, entitled "Absorbent Article With Elastic Waist Feature and Enhanced Absorbency" issued to Alemany et al., on August 10, 1993; and in U.S. Patent No. 5,147,345, entitled "High Efficiency Absorbent Articles For Incontinence Management" issued to Young, LaVon and Taylor on September 15, 1992. In a preferred embodiment, the acquisition/distribution core 84 includes chemically treated stiffened cellulosic fiber material, available from Weyerhaeuser Co. (U.S.A.) under the trade designation of "CMC". Preferably, the acquisition/distribution core 84 has a basis weight of from 40 g/m² to 400 g/m², more preferably from 75 g/m² to 300 g/m².

More preferably, the chassis 22 further includes an acquisition/distribution layer 82 between the topsheet 24 and the acquisition/distribution core 84 as shown in Fig. 3. The acquisition/distribution layer 82 is provided to help reduce the tendency for surface wetness of the topsheet 24. The acquisition/distribution layer 82 preferably includes carded, resin bonded hiloft nonwoven materials such as, for example, available as Code No. FT-6860 from Polymer Group, Inc., North America (Landisiville, New Jersey, U.S.A.), which is made of polyethylene telephthalate fibers of 6 dtex, and has a basis weight of about 43 g/m². A preferable example for the acquisition/distribution layer 82 and the acquisition/distribution core 84 is disclosed in EP 0797968A1 (Kurt et al.) published on October 1, 1997.

The topsheet 24 is preferably compliant, soft feeling, and non-irritating to the wearer's skin. Further, the topsheet 24 is liquid pervious permitting liquids (e.g., urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 25 (i.e., to prevent rewet). If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 25. The topsheet 24 can be rendered hydrophilic by treating it with a surfactant. Suitable methods for treating the topsheet 24 with a surfactant include spraying the topsheet 24 material with the surfactant and immersing the material into the surfactant. A more detailed discussion of such a treatment and hydrophilicity is contained in U.S. Patent No. 4,988,344 entitled "Absorbent Articles with Multiple Layer Absorbent Layers" issued to Reising, et al. on January 29, 1991 and U.S. Patent No. 4,988,345 entitled "Absorbent Articles with Rapid Acquiring Absorbent Cores" issued to Reising on January 29, 1991.

In a preferred embodiment, the topsheet 24 is a nonwoven web that can provide reduced tendency for surface wetness; and consequently facilitate maintaining urine absorbed by the core 25 away from the user's skin, after wetting. One of the preferred topsheet materials is a thermobonded carded web which is available as Code No. P-8 from Fiberweb North America, Inc. (Simpsonville, South Carolina, U.S.A.). Another preferred topsheet material is available as Code No. S-2355 from Havix Co., Japan. This material is a bi-layer composite material, and made of two kinds of synthetic surfactant treated bicomponent fibers by using carding and air-through technologies. Yet another preferred topsheet material is a thermobonded carded web which is available as Code No. Profleece Style 040018007 from Amoco Fabrics, Inc. (Gronau, Germany).

Another preferred topsheet 24 includes an apertured formed film. Apertured formed films are preferred for the topsheet 24 because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent No. 3,929,135, entitled "Absorptive Structures Having Tapered Capillaries", issued to Thompson on December 30, 1975; U.S. Patent No. 4,324,246 entitled "Disposable Absorbent Article Having A Stain Resistant Topsheet", issued to Mullane, et al. on April 13, 1982; U.S. Patent No. 4,342,314 entitled "Resilient Plastic Web Exhibiting Fiber-Like Properties", issued to Radel. et al. on August 3, 1982; U.S. Patent No. 4,463,045 entitled "Macroscopically Expanded Three-Dimensional Plastic Web Exhibiting Non-Glossy Visible Surface and Cloth-Like Tactile Impression", issued to Ahr et al. on July 31, 1984; and U.S. 5,006,394 "Multilayer Polymeric Film" issued to Baird on April 9, 1991.

In a preferred embodiment, the backsheet 22 includes the liquid impervious film 68 as shown in, for example, Fig. 3. Preferably, the liquid impervious film 68 longitudinally extends in the front, back and crotch regions 26, 28 and 30. More preferably, the liquid impervious film 68 does not laterally extend into the at least one of the ear panels 46 or 48. The liquid impervious film 68 has a body-facing surface 79 and an outer-facing surface 77 opposing the body-facing surface 79. The liquid impervious film 68 is impervious to liquids (e.g., urine) and is preferably manufactured from a thin plastic film. However, more preferably the plastic film permits vapors to escape from the garment 20. In a preferred embodiment, a microporous polyethylene film is used for the liquid impervious film 68. A suitable microporous polyethylene film is manufactured by Mitsui Toatsu Chemicals, Inc., Nagoya, Japan and marketed in the trade as PG-P. In a preferred embodiment, a disposable tape (not shown in Figs.) is additionally joined to the outer surface of the backsheet 22 to provide a convenient disposal after soiling. A preferred disposal tape (or device) for pull-on garments is disclosed in International Publication No. WO 94/09736 (Rollag et al.) published on May 11, 1994.

A suitable material for the liquid impervious film 68 is a thermoplastic film having a thickness of from 0.012 mm (0.5 mil) to 0.051 mm (2.0 mils), preferably including polyethylene or polypropylene. Preferably, the liquid impervious film has a basis weight of from 5 g/m² to 35 g/m². However, it should be noted that other flexible liquid impervious materials may be used.

The backsheet 22 further includes the outer cover nonwoven layer 74 (i.e., the chassis layer 40) which is joined with the outer-facing surface 77 of the liquid impervious film 68 to form a laminate. The outer cover nonwoven layer 74 is formed by the spunbonded nonwoven layer of the polypropylene/polyethylene copolymer of the present invention. The outer cover nonwoven layer 74 preferably covers all of the outer-facing surface of the pull-on garment 20 to provide the feel and appearance of a cloth garment. The outer cover nonwoven layer 74 may be joined to the liquid impervious film 68 by any suitable attachment means known in the art. For example, the outer cover nonwoven layer 74 may be secured to the liquid impervious film 68 by a uniform continuous layer of adhesive, a patterned layer of adhesive, or an array of separate lines, spirals, or spots of adhesive. Suitable adhesives include a hotmelt adhesive obtainable from Nitta Findley Co., Ltd., Osaka, Japan as H-2128, and a hotmelt adhesive obtainable from H.B. Fuller Japan Co., Ltd., Osaka, Japan as JM-6064.

In a preferred embodiment, the outer cover nonwoven layer 74 is a spunbonded nonwoven web of polypropylene/polyethylene copolymer, for example, obtainable from Mitsui Chemical Co., Ltd., Tokyo, Japan, under Code No. 23GSM. The outer cover nonwoven layer 74 is formed from component fibers of the polypropylene/polyethylene copolymer.

The backsheet 22 is positioned adjacent the outer-facing surface of the absorbent core 25 and is preferably joined thereto by any suitable attachment means known in the art. Specifically, the body-facing surface 79 of the liquid impervious film 68 may be secured to the absorbent core 25 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota, U.S.A., and marketed as HL-1358J. An example of a suitable attachment means including an open pattern network of filaments of adhesive is disclosed in U.S. Patent No. 4,573,986 entitled "Disposable Waste-Containment Garment", which issued to Minetola et al. on March 4, 1986. Another suitable attachment means including several lines of adhesive filaments swirled into a spiral pattern is illustrated by the apparatus and methods shown in U.S. Patent No. 3,911,173 issued to Sprague, Jr. on October 7, 1975; U.S. Patent No. 4,785,996 issued to Ziecker, et al. on November 22, 1978; and U.S. Patent No. 4,842,666 issued to Werenicz on June 27, 1989. Alternatively, the attachment means may include heat bonds, pressure bonds, ultrasonic bonds, dynamic mechanical bonds, or any other suitable attachment means or combinations of these attachment means as are known in the art.

In an alternative embodiment, the absorbent core 25 is not joined to the backsheet 22, and/or the topsheet 24 in order to provide greater extensibility in the front region 26 and the back region 28.

The pull-on garment 20 preferably further includes elasticized leg cuffs 52 for providing improved containment of liquids and other body exudates. The elasticized leg cuffs 52 may include several different embodiments for reducing the leakage of body exudates in the leg regions. (The leg cuffs can be and are sometimes also referred to as leg bands, side flaps, barrier cuffs, elastic cuffs or gasketing cuffs.) U.S. Patent 3,860,003 entitled "Contractable Side Portions for Disposable Diaper" issued to Buell on January 14, 1975, describes a disposable diaper which provides a contractible leg opening having a side flap and one or more elastic members to provide an elasticized leg cuff. U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987; and U.S. Patent 4,795,454 entitled "Absorbent Article Having Leakage-Resistant Dual Cuffs" issued to Dragoo on January 3, 1989, describe disposable diapers having dual cuffs including a gasketing cuff and a barrier cuff. U.S. Patent 4,704,115 entitled "Disposable Waist Containment Garment" issued to Buell on November 3, 1987, discloses a disposable diaper or incontinence garment having side-edge-leakage-guard gutters configured to contain free liquids within the garment.

While each elasticized leg cuff 52 may be configured so as to be similar to any of the leg bands, side flaps, barrier cuffs, or elastic cuffs described above, it is preferred that the elasticized leg cuff 52 includes an elastic gasketing cuff 62 with one or more elastic strands 64 as shown in Fig. 2, which is described in the above-referred U.S. Patent Nos. 4,695,278 and 4,795,454. It is also preferred that each elasticized leg cuff 52 further includes inner barrier cuffs 54 each including a barrier flap 56 and a spacing means 58 which are described in the above-referenced U.S. Patent No. 4,909,803.

The pull-on garment 20 preferably further includes an elasticized waistband 50 that provides improved fit and containment. The elasticized waistband 50 is that portion or zone of the pull-on garment 20 which is intended to elastically expand and contract to dynamically fit the wearer's waist. The elasticized waistband 50 preferably extends longitudinally outwardly from the waist edge of the pull-on garment 20 toward the waist edge of the absorbent core 25. Preferably, the pull-on garment 20 has two elasticized waistbands 50, one positioned in the back region 28 and one positioned in the front region 26, although other pull-on garment embodiments can be constructed with a single elasticized waistband. The elasticized waistband 50 may be constructed in a number of different configurations including those described in U.S. Patent 4,515,595 entitled "Disposable Diapers with Elastically Contractible Waistbands" issued to Kievit et al. on May 7, 1985 and the above referenced U.S. Patent 5,151,092 issued to Buell.

In a preferred embodiment, the waistbands 50 includes the elastic laminate of the present invention.

The waistbands 50 may include materials that have been "prestrained" or "mechanically prestrained" (i.e., subjected to some degree of localized pattern mechanical stretching to permanently elongate the material). The materials may be prestrained using deep embossing techniques as are known in the art. Alternatively, the materials may be prestrained by directing the material through an incremental mechanical stretching system as described in U.S. Patent No. 5,330,458 entitled "Absorbent Article With Elastic Feature Having A Portion Mechanically Prestrained" issued to Buell et al., on July 19, 1994. The materials are then allowed to return to their substantially untensioned condition, thus forming a "zero strain" stretch laminate that is extensible, at least up to the point of initial stretching.

At least one pair of the ear panels 46 and 48 includes the elastic member 70 as shown in Fig. 3. The elastic member 70 of the front ear panels 46 includes an elastomeric material layer 124 (not shown in Fig. 3) which preferably extends laterally outward from the chassis 41 to provide good fitness by generating the optimal retention (or sustained) force at the waist and side areas of the wearer. Preferably, the elastomeric material layer 124 is extensible in at least one direction, preferably in the lateral direction to generate a retention (or sustained) force that is optimal to prevent the pull-on garment 20 from drooping, sagging, or sliding down from its position on the torso without causing the red marking on the skin of the wearer. In a preferred embodiment, each of the ear panels 46 and 48 includes the elastomeric material layer 124.

The elastic member 70 is operatively joined to the outer cover nonwoven layer 74 and preferably the nonwoven webs 72 in the ear panels 46 and 48 to form a laminate. In a preferred embodiment; the elastic member 70 is operatively joined to the nonwoven webs 72 and 74 by securing them to at least one, preferably both of the nonwoven webs 72 and 74 while in a substantially untensioned (zero strain) condition.

The elastic member 70 can be operatively joined to the nonwoven webs 72 and 74, by using either an intermittent bonding configuration or a substantially continuous bonding configuration. Herein, "intermittently" bonded laminate web means a laminate web wherein the plies are initially bonded to one another at discrete spaced apart points or a laminate web wherein the plies are substantially unbonded to one another at discrete spaced apart areas. Conversely, a "substantially continuously" bonded laminate web means a laminate web wherein the plies are initially bonded substantially continuously to one another throughout the areas of interface. It is preferred that the stretch laminate be bonded over all or a significant portion of the stretch laminate so that the inelastic webs (i.e., the nonwoven webs 72 and 74) elongate or draw without causing rupture, and the layers of the stretch laminates are preferably bonded in a configuration that maintains all of the layers of the stretch laminate in relatively close adherence to one another after the incremental mechanical stretching operation. Consequently, the elastic panel members and the other plies of the stretch laminate are preferably substantially continuously bonded together using an adhesive. In a particularly preferred embodiment, the adhesive selected is applied with a control coat spray pattern at a basis weight of about 7.0 grams/m². The adhesive pattern width is about 6.0 cm. The adhesive is preferably an adhesive such as is available from Nitta Findley Co., Ltd., Osaka, Japan, under the designation H2085F. Alternatively, the elastic panel member and any other components of the stretch laminates may be intermittently or continuously bonded to one another using heat bonding, pressure bonding, ultrasonic bonding, dynamic mechanical bonding, or any other method as is known in the art.

After the elastic member 70 is operatively joined to the nonwoven webs 72 and 74, at least a portion of the resultant composite stretch laminate is then subjected to mechanical stretching sufficient to permanently elongate the non-elastic components which are, for example, the nonwoven webs 72 and 74. The composite stretch laminate is then allowed to return to its substantially untensioned condition. At least one pair of, preferably both of the ear panels 46 and 48 is thus formed into "zero strain" stretch laminates. This configuration allows the ear panels 46 and 48 to be elastically extensible in at least the lateral direction.

The elastic member 70 is preferably joined to, more preferably directly secured to the respective edges 78 of the liquid impervious film (i.e., the liquid impervious film 68) through an adhesive 76 as shown in Fig. 3. In a preferred embodiment, while liquid impervious film 68 longitudinally extends in the front, back and crotch regions 26, 28 and 30, it does not laterally extend into at least one of, preferably each of the extensible ear panels 46 and 48. In a more preferred embodiment, the elastic member 70 is joined to the respective edges 78 of the liquid impervious film 68 at the outer-facing surface 77 as shown in Fig. 3. In an alternative embodiment, the elastic member 70 may be joined to the respective edges 78 of the liquid impervious film 68 at the body-facing surface 79 (not shown in Figs.). Preferably, the adhesive 76 is applied in a spiral glue pattern. In a preferred embodiment, the adhesive 76 is a flexible adhesive with an amorphous and crystallizing component. Such a preferred adhesive is made by Nitta Findley Co., Ltd., Osaka, Japan, under the designation H2085F. Alternatively, the elastic member 70 may be joined to the respective edges 78 of the liquid impervious film 68 by any other bonding means known in the art which include heat bonds, pressure bonds, ultrasonic bonds, dynamic mechanical bonds, or combinations of these attachment means.

Fig. 4 is a cross-sectional view of an elastic member (or an elastic laminate) of one preferred embodiment of the present invention. Referring to Fig. 4, the elastic member 70 includes the elastomeric material layer 124 having a first surface 150 and a second surface 152 opposing the first surface 150, and a first coverstock layer 122 which is joined to the first surface 150 of the elastomeric material layer 124. In a preferred embodiment, the first coverstock layer 122 is joined to the first surface 150 of the elastomeric material layer 124 by an adhesive (not shown in Fig. 4). More preferably, the elastic member 70 further includes a second coverstock layer 126 which is joined to the second surface 152 of the elastomeric material layer 124 by an adhesive (not shown in Fig. 4).

Preferably, the elastic member 70 is joined to the respective edges 78 of the liquid impervious film 68 at the outer-facing surface 77 as shown in Fig. 3. In an alternative embodiment, the elastic member 70 may be joined to the respective edges 78 of the liquid impervious film 68 at the body-facing surface 79 (not shown in Figs.).

The elastomeric material layer 124 may be formed in a wide variety of sizes, forms and shapes. In a preferred embodiment, the elastomeric material layer 124 is in the form of a continuous plane layer. Preferred forms of continuous plane layer include a scrim, a perforated (or apertures formed) film, an elastomeric woven or nonwoven, and the like. The continuous plane layer may take any shape which can be suitably provided in the ear panels. Preferred shapes of continuous plane layer include a quadrilateral including a rectangle and a square, a trapezoid, and the other polygons. In an alternative embodiment, the elastomeric material layer 124 is in the form of discrete strands (or strings) which are not connected each other.

Elastomeric materials which have been found to be especially suitable for the elastomeric material layer 124 are styrenic block copolymer based scrim materials, perforated (or apertured) elastic films, preferably with a thickness of from about 0.05 mm to about 1.0 mm (0.002 inch - 0.039 inch). Other suitable elastomeric materials for the elastomeric material layer 124 include "live" synthetic or natural rubber, other synthetic or natural rubber foams, elastomeric films (including heat shrinkable elastomeric films), elastomeric woven or nonwoven webs, elastomeric composites, or the like.

In the preferred embodiment shown in Fig. 4, the elastomeric scrim 124 has a plurality of first strands 125 and a plurality of second strands 127. The plurality of first strands 125 intersect the plurality of second strands 127 at nodes 130 at a predetermined angle α, forming a net-like open structure having a plurality of apertures 132. Each aperture 132 is defined by at least two adjacent first strands and at least two adjacent second strands, so that the apertures 132 are substantially rectangular in shape. Other configurations of the apertures 132, such as parallelograms, squares, or circular arc segments, can also be provided. Preferably, the first and second strands 125 and 127 are substantially straight and substantially parallel to one another. Preferably, the first strands 125 intersect the second strands 127 at nodes 130 such that the angle α is about 90 degrees. The first and second strands 125 and 127 are preferably joined or bonded at nodes 90.

A preferred elastomeric scrim 124 is manufactured by the Conwed Plastics Company (Minneapolis, Minn., U.S.A.) under the designation XO2514. This material has about 12 elastic strands per inch in the structural direction B (i.e., the first strands 125) and about 7 elastic strands per inch in the structural direction D (i.e., the second strands 127).

In an alternative preferred embodiment, the elastomeric material layer 124 is a porous, macroscopically-expanded, three-dimensional elastomeric web which was already described by referring to Fig. 5.

In the embodiment shown in Fig. 4, the elastic member 70 includes first and second coverstock layers 122 and 126, and elastomeric material layer 124 disposed in the first and second coverstock layers 122 and 126. The first coverstock layer 122 has an inner surface 142 and an outer surface 144. The inner surface 142 of the first coverstock layer 122 is the surface that is positioned facing the elastomeric material layer 124. The second coverstock layer 126 also has an inner surface 146 and an outer surface 148. The inner surface 146 of the second coverstock layer 126 is the surface that is positioned facing the elastomeric material layer 124. The elastomeric material layer 124 also has two planar surfaces, first surface 150 and second surface 152, each of which is substantially parallel with the planes of the first and second coverstock layers 122 and 126. The first surface 150 is that planar surface of the elastomeric material layer 124 that is most closely adjacent with the inner surface 142 of first coverstock layer 122. The second surface 152 is that planar surface of elastomeric material layer 124 that is most closely adjacent to the inner surface 146 of the second coverstock layer 126.

Since the elastic member 70 will be subjected to mechanical stretching before and during use, the first and second coverstock layers 122 and 126 need to have a relatively high elongation at breaking, and are more preferably stretchable or elongatable, yet more preferably drawable (but not necessarily elastomeric), without undue (and preferably without any), tearing or ripping. Further, the first and second coverstock layers 122 and 126 are preferably compliant, soft feeling, and non-irritating to the wearer's skin and give the article the feel and comfort of a cloth garment.

An exemplary preferred nonwoven material for the first and second coverstock layers 122 and 126 is a consolidated nonwoven material available from the Fiberweb North America, Inc. (Simpsonville, South Carolina, U.S.A.) under the designation Sofspan 200. Herein, "consolidated nonwoven material" means a nonwoven material that has been gathered or necked under mechanical tension in the structural direction D so that the material can elongate in the structural direction D under low force. This material has a basis weight of 25 g/m² before consolidation and a basis weight of about 63g/m2 after consolidation. In an alternative preferred embodiment, the spunbonded nonwoven of polypropylene/polyethylene copolymer of the present invention may be used for the first and second coverstock layers 122 and 126.

The elastomeric material layer 124 and the first and second coverstock layers 122 and 126 are joined together, preferably by using an adhesive, to form the elastic member 70. A preferred method for making the elastic member 70 is described in International Publication No. WO 98/55298 (Langdon et al.) published on December 10, 1998.

It is understood that the examples and embodiments described herein are for illustrative purpose only and that various modifications or changes will be suggested to one skilled in the art without departing from the scope of the present invention.

## Claims

1. An elastic laminate elastically extensible in at least one direction, comprising:
an elastic member having a first surface and a second surface opposing the first surface; and
a spunbonded nonwoven layer joined to the first surface of the elastic member to form a laminate, the spunbonded nonwoven layer including component fibers formed by a polypropylene/polyethylene copolymer;
the laminate being mechanically stretched to form an elastic laminate, wherein the spunbonded nonwoven layer has a Young's modulus of greater than (1575 N/cm 4000 gf/inch), and wherein the spunbonded nonwoven layer has a stress of less than (394 N/cm 1000 gf/inch) at peak in the Stress-Strain curve.

2. The elastic laminate of Claim 1, wherein the component fibers have an average fiber denier of less than 2.2.

3. The elastic laminate of Claim 1, wherein the elastic member includes an elastomeric material which has plurality of apertures formed therein.

4. The elastic laminate of Claim 1, wherein the copolymer is a random or block polypropylene/polyethylene copolymer.

5. The elastic laminate of Claim 4, wherein the copolymer is a polypropylene modified by copolymerizing from 0.5% to 20% of polyethylene randomly in the backbone.

6. A disposable article comprising the elastic laminate of Claim 1.

7. The disposable article of Claim 6, wherein the disposable article is a disposable garment including at least one pair of extensible ear panels, and wherein at least one of the side panels includes the elastic laminate of Claim 1.

8. The disposable garment of Claim 6, wherein the disposable article is a disposable garment including a waistband, and wherein the waistband includes the elastic laminate of Claim 1.

## Patentansprüche

1. Elastischer Schichtstoff, der in mindestens eine Richtung elastisch dehnbar ist, umfassend:
ein Elastikelement mit einer ersten Oberfläche und einer zweiten Oberfläche, die der ersten Oberfläche gegenüber liegt; und
eine Spinnvliesschicht, die mit der ersten Oberfläche des Elastikelements verbunden ist, um einen Schichtstoff zu bilden, wobei die Spinnvliesschicht Komponentenfasern umfasst, die durch ein Polypropylen/Polyethylen-Copolymer gebildet werden;
wobei der Schichtstoff mechanisch gedehnt wird, um einen elastischen Schichtstoff zu bilden, worin die Spinnvliesschicht einen Youngschen Elastizitätsmodul von über 1575 N/cm (4000 p/Inch) aufweist und worin die Spinnvliesschicht eine Spannung von unter 394 N/cm (1000 p/Inch) an der Spitze in der Spannung-Dehnung-Kurve aufweist.

2. Elastischer Schichtstoff nach Anspruch 1, wobei die Komponentenfasern ein durchschnittliches Faser-Denier von unter 2,2 aufweisen.

3. Elastischer Schichtstoff nach Anspruch 1, wobei das Elastikelement ein elastomeres Material mit mehreren darin geformten Öffnungen umfasst.

4. Elastischer Schichtstoff nach Anspruch 1, wobei das Copolymer ein statistisches Polypropylen/Polyethylen-Copolymer oder Polypropylen/Polyethylen-Blockcopolymer ist.

5. Elastischer Schichtstoff nach Anspruch 4, wobei das Copolymer ein Polypropylen ist, das durch statistische Copolymerisation von 0,5 % auf 20 % Polyethylen in der Hauptkette modifiziert wurde.

6. Einwegartikel, umfassend den flexiblen Schichtstoff nach Anspruch 1.

7. Einwegartikel nach Anspruch 6, wobei der Einwegartikel ein Einwegkleidungsstück ist, das mindestens ein Paar dehnbare Lappenfelder umfasst, und wobei mindestens eines der Seitenfelder den elastischen Schichtstoff nach Anspruch 1 umfasst.

8. Einwegkleidungsstück nach Anspruch 6, wobei der Einwegartikel ein Einwegkleidungsstück ist, das ein Taillenband umfasst, und wobei das Taillenband den elastischen Schichtstoff nach Anspruch 1 umfasst.

## Revendications

1. Stratifié élastique élastiquement extensible dans au moins une direction, comprenant:
un élément élastique ayant une première surface et une deuxième surface opposée à la première surface; et
une couche non-tissée filée-liée attachée à la première surface de l'élément élastique pour former un stratifé, la couche non-tissée incluant des fibres composantes formées par un copolymère de polypropylène/polyéthylène;
le stratifié étant mécaniquement étiré de façon à former un stratifié élastique, dans lequel la couche non-tissée filée-liée a un module d'Young supérieur à 1575 N/cm (4000 gf/pouce), et dans lequel la couche non-tissée filée-liée a une contrainte de moins de 394 N/cm (1000 gf/pouce) au niveau du pic sur la courbe Contrainte-Déformation.

2. Stratifié élastique de la Revendication 1, dans lequel les fibres composantes ont un denier moyen de fibre inférieur à 2,2.

3. Stratifié élastique de la Revendication 1, dans lequel l'élément élastique inclut un matériau élastomère qui a une pluralité d'orifices formés dans celui-ci.

4. Stratifié élastique de la Revendication 1, dans lequel le copolymère est un copolymère de polypropylène/polyéthylène aléatoire ou séquencé.

5. Stratifié élastique de la Revendication 4, dans lequel le copolymère est un polypropylène modifié par copolymérisation à partir de 0,5 % à 20 % de polyéthylène au hasard dans le squelette.

6. Article jetable comprenant le stratifié élastique de la Revendication 1.

7. Article jetable de la Revendication 6, dans lequel l'article jetable est un vêtement jetable incluant au moins une paire de pans à oreilles extensibles, et dans lequel au moins un des pans latéraux inclut le stratifié élastique de la Revendication 1.

8. Vêtement jetable de la Revendication 6, dans lequel l'article jetable est un vêtement jetable incluant une ceinture, et dans lequel la ceinture inclut le stratifié élastique de la Revendication 1.
